# EUROPEAN PATENT SPECIFICATION

(11) **EP 2 546 653 B1**
(45) Date of publication and mention of the grant of the patent: **15.04.2015**
(21) Application number: 12176052.4
(22) Date of filing: 12.07.2012
(51) Int. Cl.: G01N 33/543, C12Q 1/00, G01N 33/72

(54) **Electrode for Measuring Glycoprotein and Preparation Method Thereof**
Elektrode zum Messen von Glykoproteinen und Herstellungsverfahren davon
Électrode de mesure de glycoprotéine et son procédé de préparation

(30) Priority: 15.07.2011 KR 20110070646
(43) Date of publication of application: 16.01.2013
(73) Proprietor: LG Electronics Inc., Yeongdeungpo-gu Seoul 150-721 (KR)
(72) Inventor: Ku, Yun Hee, 137-724 Seoul (KR); Yang, Yong Ju, 150-721 Seoul (KR); Park, Yoo Min, 443-749 Gyeonggi-do (KR); Song, Seung Yeon, 443-749 Gyeonggi-do (KR); Han, Yong Duk, 443-749 Gyeonggi-do (KR); Kim, Moo Sub, 150-721 Seoul (KR); Yoon, Hyun Chul, 443-749 Gyeonggi-do (KR)
(74) Representative: Cabinet Plasseraud

(56) References cited:
- US-A1- 2003 148 169
- KITANO H ET AL: "Accumulation of phenyl boronic acid-carrying telomers on a gold surface", JOURNAL OF COLLOID AND INTERFACE SCIENCE, ACADEMIC PRESS, NEW YORK, NY, US, vol. 273, no. 1, 1 May 2004 (2004-05-01), pages 106-114, XP027132328, ISSN: 0021-9797 [retrieved on 2004-04-02]

## Description

### BACKGROUND OF THE INVENTION

### Field of the Invention

The present invention relates to an electrode for measuring glycoprotein, and more particularly, to an electrode for measuring glycoprotein, the surface of which can bind an increased amount of boronic acid capable of binding to glycoprotein and which can more accurately measure the amount of glycoprotein, and to a preparation method thereof.

### Description of the Prior Art

Blood glucose and glycated hemoglobin (HbA1c) are most frequently used as important indexes that reflect the current status of diabetes. Particularly, in the case of glycated hemoglobin, a test error caused by an empty stomach and a drug is low and it is possible to predict long-term changes in the blood glucose levels of diabetic patients before blood collection. Thus, the measurement of glycated hemoglobin is useful for assessment of the long-term regulation of plasma glucose. In addition, glycated hemoglobin has good specificity, and thus is considered as a good index for diabetes and related complications.

In the prior art, in order to electrochemically measure glycated hemoglobin, the electrode surface was modified with boronic acid (BA) capable of binding specifically to glycated hemoglobin. Boronic acid is frequently used in sensors for measuring glycoprotein or glucose, because it has the property of binding specifically to sugars and glycoprotein by interaction with the cis-diol. Specifically, a boronic acid group is exposed to the electrode surface to which glycated hemoglobin is then immobilized, and the concentration of the glycated hemoglobin is measured in the resulting change in electrochemical signals.

For example, Korean Patent Laid-Open Publication No. 2011-0002293 discloses preparing an electrode by binding a dendrimer to the electrode surface and then binding 4-formyl-phenylboronic acid thereto, and also discloses reacting glycated hemoglobin with the 4-formyl-phenylboronic acid and then electrochemically measuring the amount of the glycated hemoglobin. However, even when the 4-formyl-phenylboronic acid is used, there are shortcomings in that the reaction of glycated hemoglobin is insufficient and the density of 4-formyl-phenylboronic acid that is bound to the electrode surface by the dendrimer is low.

### SUMMARY OF THE INVENTION

Accordingly, the present invention has been made in view of the above-described problems occurring in the prior art, and it is an object of the present invention to increase the amount of boronic acid that is bound to the electrode surface and to more accurately measure the amount of glycoprotein.

Another object of the present invention is to prepare an electrode having a boronic acid bound thereto in a simple and easy manner.

To achieve the above objects, the present invention provides an electrode for measuring glycoprotein as defined in claim 1. Cystamine (Cys) is bound to the surface of the electrode, and boronic acid (BA) is conjugated to the cystamine.

Herein, the boronic acid is preferably formylphenyl boronic acid (FPBA), and more preferably a 3-formylphenyl boronic acid (3-FPBA) represented by the following formula 1:

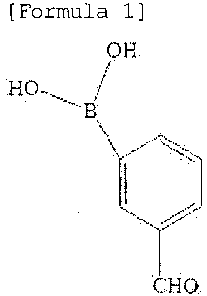

Moreover, in the electrode of the present invention, two 3-formylphenyl boronic acids may be conjugated to one cystamine.

Furthermore, the cystamine may be bound to the electrode surface by the disulfide group of the cystamine.

In one embodiment, the present invention provides an electrode for measuring glycoprotein, wherein a cystamine-boronic acid conjugate prepared by conjugating cystamine to boronic acid is bound to the electrode surface.

Herein, the conjugate may be bound to the electrode surface by the disulfide group of the cystamine.

Moreover, the conjugate may be Cys-FPBA₂ represented by the following formula 2:

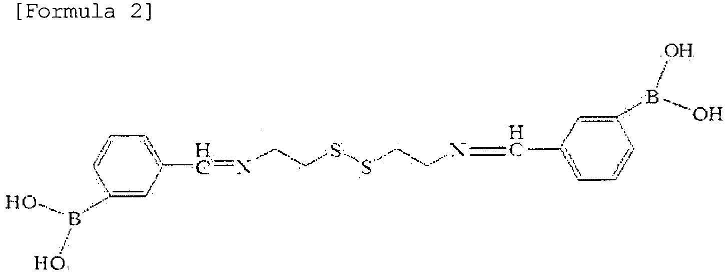

In addition, the electrode surface may be made of gold (Au).

In another aspect, the present invention provides a method for preparing an electrode for measuring glycoprotein as defined in claim 10. The method includes the steps of: preparing a cystamine-boronic acid conjugate by conjugating cystamine to boronic acid; and binding the prepared conjugate to the surface of an electrode.

Herein, the step of preparing the conjugate may include a step of reacting the cystamine with the boronic acid for 3-5 hours.

Furthermore, the step of preparing the conjugate may also include a step of reacting the cystamine with the boronic acid in the presence of triethylamine (TEA).

In addition, the step of preparing the conjugate preferably includes a step of reacting the cystamine with the boronic acid at a temperature of 40∼60 °C.

Details of other embodiments of the present invention are included in the following detailed description and the accompanying drawings.

### BRIEF DESCRIPTION OF THE DRAWINGS

FIG. 1 is a schematic diagram showing a process of preparing a glycoprotein-measuring electrode according to one embodiment of the present invention, and a method of measuring glycoprotein using the electrode.
FIG. 2 shows electrochemical signals (current) measured on an electrode prepared by each of a bottom-up method (●, red line) and a conjugation method (▲, blue line) according to one embodiment of the present invention.
FIG. 3 shows electrochemical signals (current) measured on Cys-FPBA₂ electrodes synthesized by reacting cystamine with boronic acid for various times according to one embodiment of the present invention.
FIG. 4 shows electrochemical signals (current) measured on Cys-FPBA₂ electrodes synthesized by reacting cystamine with boronic acid in the presence of various catalysts according to one embodiment of the present invention.
FIG. 5 shows electrochemical signals (current) measured on Cys-FPBA₂ electrodes synthesized by reacting cystamine with boronic acid at various temperatures. In FIG. 5, Cys-FPBA₂_25 (●, red line) : reacted at 25 °C; and Cys-FPBA₂ _50 (▲, blue line) : reacted at 50 °C.
FIG. 6 shows the results of carrying out a glucose oxidase (GOX) immobilization test on an electrode having a cystamine-boronic acid conjugate bound thereto according to one embodiment of the present invention (▲, blue line) and an electrode having a cystamine bound thereto according to a comparative example (▲, blue line).
FIG. 7 shows electrochemical signals (current) and COV values as a function of the concentration of an HbA1c sample, measured using an electrode according to one embodiment of the present invention.
FIG. 8 shows electrochemical signals (current) and COV values as a function of the concentration (%) of HbA1c, measured using an electrode according to one embodiment of the present invention.
FIG. 9 shows electrochemical signals (current) and COV values as a function of the concentration (%) of HbA1c in reference samples, measured using an electrode according to one embodiment of the present invention.

### DETAILED DESCRIPTION OF THE INVENTION

Although the present invention can be modified variously and have several embodiments, exemplary embodiments are illustrated in the accompanying drawings and will be described in detail in the detailed description. However, the present invention is not limited to the specific embodiments and should be construed as including all the changes, equivalents and substitutions included in the spirit and scope of the present invention. In the following description, the detailed description of related known technology will be omitted when it may obscure the subject matter of the present invention.

Terms used in this specification are used only to describe a specific embodiment and are not intended to limit the scope of the present invention. Singular expressions include plural expressions unless specified otherwise in the context thereof. In this specification, the terms "comprise", "have", etc., are intended to denote the existence of mentioned characteristics, numbers, steps, operations, components, parts, or combinations thereof, but do not exclude the probability of existence or addition of one or more other characteristics, numbers, steps, operations, components, parts, or combinations thereof.

The present invention is directed to an electrode for measuring glycoprotein, and more particularly to an electrode capable of detecting the presence or measuring the amount of a glycoprotein having the cis-diol group of the carbohydrate chain, for example, glycated hemoglobin (HbA1c) or glycated albumin.

The electrode that is used in the present invention may have a conductive electrode body, for example made of gold (Au) or platinum (Pt) electrode, or consisting of a semiconductor electrode such as a carbon electrode. Preferably, the gold electrode is used.

In order for the inventive electrode to bind to a glycated protein having the cis-diol group of the carbohydrate chain, the electrode body preferably has on its surface a boronic acid (BA) capable of binding the cis-diol group by interaction with the cis-diol. In order to bind the boronic acid to the electrode surface, a cystamine (Cys) is present between the electrode and the boronic acid. Herein, the boronic acid is not specifically limited and may be any boronic acid which is widely known in the art. For example, the boronic acid may be formylphenyl boronic acid (FPBA), preferably 3-formylphenyl boronic acid (3-FPBA), and more preferably a 3-formylphenyl boronic acid (3-FPBA) represented by formula 1.

Specifically, in the inventive electrode for measuring glycoprotein, the cystamine is bound to the electrode surface, and the boronic acid is conjugated to the cystamine. The cystamine includes a disulfide group which can bind to the electrode surface made of, for example, a metal, and it has an amine group at both ends, and thus can also bind to a boronic acid having an aldehyde group. Thus, one cystamine can be conjugated to two 3-formylphenyl boronic acids by amino groups at both ends.

In addition, in order to more effectively bind the cystamine, the electrode of the present invention may further comprise a surface modification layer which is known to those skilled in the art.

In the prior art, in order to bind boronic acid to the electrode surface, 3,3'-dithio-bis-propionic acid N-hydroxysuccinimide ester (DTSP) having a functional group capable of reacting with an amine was bound to the electrode surface, and a dendrimer such as poly(amidoamine) or poly(alkyleneimine) was bound thereto, and then boronic acid was bound to the dendrimer. In other words, a structure such as BA/dendrimer/DTSP/electrode was prepared.

However, according to the present invention, cystamine is bound to the electrode surface, and boronic acid is bound directly thereto. Thus, a structure of BA/cystamine/electrode which is simpler than the prior art structure is provided, and the preparation method is simpler and easier than the prior art.

This electrode of the present invention may be prepared by binding a cystamine to the electrode surface and conjugating a boronic acid to the cystamine. Specifically, this electrode structure is preferably prepared by a conjugation method in which a cystamine is conjugated with a boronic acid to prepare a Cys-FPBA₂ conjugate which is then bound to the electrode surface by dipping the electrode once in a solution containing the conjugate. Alternatively, the electrode structure may also be prepared by a bottom-up method in which a cystamine is bound to the electrode surface and then a boronic acid is bound to the cystamine.

The inventive electrode structure consisting of boronic acid/cystamine/electrode surface could not be conceived of from the prior art. The reason is as follows. First, the disulfide group of cystamine is not located at the end of the cystamine, but is located at the middle of the cystamine. Second, the electrode having the boronic acid bound thereto is preferably prepared by the conjugation method compared to the bottom-up method which is a general method.

Thus, in one embodiment, the present invention provides an electrode for measuring glycoprotein, in which a cystamine-boronic acid conjugate is bound to the electrode surface. Herein, the conjugate may be a Cys-FPBA₂ synthesized by reacting cystamine with 3-formylphenyl boronic acid (3-FPBA) according to the following reaction scheme:

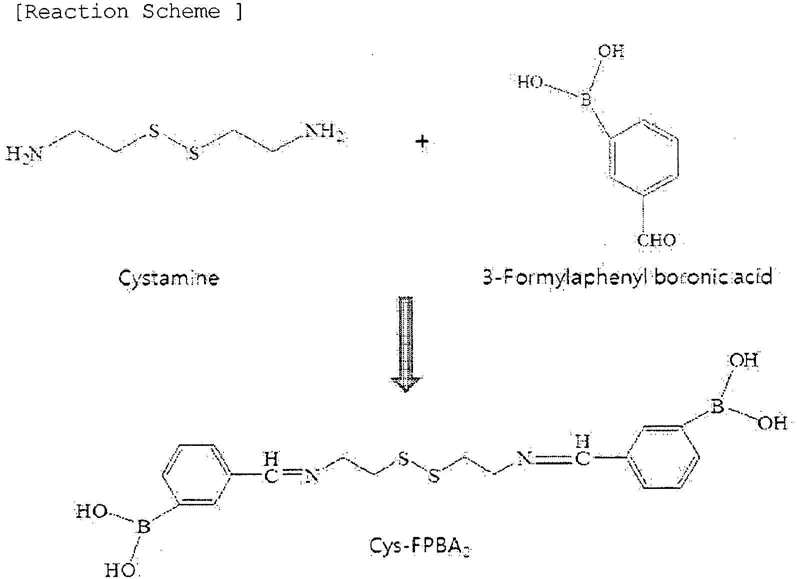

Moreover, the conjugate is preferably bound to the electrode surface such that the diol group of the boronic acid faces outward so as to bind to glycoprotein. Thus, the conjugate is preferably bound to the electrode surface by the disulfide of the cystamine.

As described above, the present invention is **characterized in that** cystamine is bound to the electrode surface and boronic acid is conjugated to the cystamine. Thus, according to the present invention, an increased amount of boronic acid can be bound to the electrode surface, so that the amount of glycoprotein can be more accurately measured.

Another aspect of the present invention is directed to a method for preparing an electrode for measuring glycoprotein, the method comprising the steps of: preparing a cystamine-boronic acid conjugate by conjugating a cystamine with a boronic acid; and binding the prepared conjugate to an electrode surface. Specifically, the electrode for measuring glycoprotein is prepared in a simple and easy manner by a conjugation method in which cystamine and boronic acid are conjugated with each other to prepare a Cys-FPBA₂ conjugate, and then an electrode body is then dipped once in a solution containing the conjugate. As described below, the electrode prepared by this method has significantly excellent effects compared to an electrode prepared by a conventional bottom-up method.

The method of preparing the conjugate is not specifically limited. For example, the cystamine can be prepared by reacting cystamine with 3-FPBA in a solvent at room temperature for several hours. Herein, the solvent is preferably DMSO (dimethyl sulfoxide).

In addition, the method of binding the conjugate is also not specifically limited. For example, the binding can be performed by washing a gold-deposited electrode surface and then dipping the electrode body in a solution of Cys-FPBA₂ in DMSO for several hours. Preferably, before the dipping process, the electrode is washed with Piranha solution to remove contaminants, and after the dipping process, the electrode is washed with DMSO and ethanol.

As described in the following examples of the present invention, the present inventors carried out tests under various conditions in order to find optimal conditions for synthesizing Cys-FPBA₂ with high yield. As a result, it was found that the reaction for preparing the conjugate is preferably carried out for 3-5 hours, and more preferably 3-4. This is because if the reaction time is shorter than 3 hours, the synthesis of Cys-FPBA₂ will be insufficient so that the signal of glucose oxidase (GOX) will be weak, and if the reaction time is longer than 5, the reaction signal decreases, rather than increases. Furthermore, the reaction is carried out in the presence of triethylamine (TEA) as a catalyst. This is because the use of triethylamine as the reaction catalyst has excellent effects compared to HCl which is frequently used as a catalyst in the general Schiff base synthesis reaction. In addition, the reaction temperature is preferably 40-60 °C, and more preferably 45-55 °C. This is because, if the reaction temperature is lower than 40 °C, the synthesis of Cys-FPBA₂ will be insufficient (the signal of glucose oxidase (GOX) will be weak), and if the reaction temperature is higher than 60 °C, the conjugate can be decomposed.

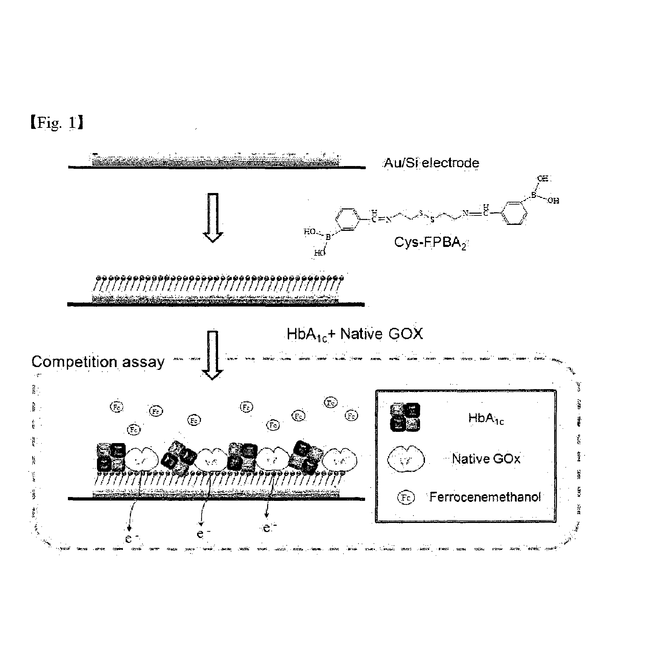

FIG. 1 is a schematic diagram showing one example of a competition assay for electrochemically measuring glycated hemoglobin (HbA1c) using an electrode for measuring glycoprotein according to one embodiment of the present invention.

As shown therein, when the electrode surface is modified with boronic acid and then various concentrations of HbA1c and a constant concentration of glucose oxidase (GOX) are simultaneously allowed to react with the electrode surface, relatively high concentrations of HbA1c interfere with the immobilization of GOX onto the electrode surface, thus reducing the electrochemical signal of GOX. Herein, GOX that is a glycoprotein can react with boronic acid even when it is not biochemically treated. When GOX is immobilized onto the electrode surface by competition with HbA1c, the amplified electrochemical signal thereof can be detected due to a biological catalytic reaction.

The present invention can be better understood by the following examples, which are merely for illustrative, not for limiting the scope of the present invention.

### Examples: Preparation of electrodes for measuring glycoprotein

To form a boronic acid self-assembled monolayer (BA SAM), the following two methods were used: a conjugation method in which a self-assembled monolayer (SAM) is formed using a synthesized cystamine/boronic acid (BA) conjugate; and a bottom-up method in which cystamine and BA are sequentially reacted with the electrode surface.

Then, in order to find optimal conditions for synthesizing Cys-FPBA₂ with high yield, tests were carried out under various conditions.

### Example 1: Preparation of glycoprotein measurement electrode by conjugation method

First, 99.999% pure gold was resistively evaporated on a titanium-treated silicon wafer (20 nm Ti) to a thickness of 200 nm, thereby preparing an electrode body.

Cystamine (5 mM) and 3-FPBA (20 mM) were allowed to react with each other in a DMSO (dimethyl sulfoxide) solvent at room temperature for 4 hours, thereby synthesizing a Cys-FPBA₂ conjugate. Herein, the amino group of the cystamine was mixed and bound to the aldehyde group of FPBA by the Schiff's base. As a catalyst, triethylamine (TEA) was added to the mixed solution before a basic atmosphere was formed.

Then, the deposited gold surface was immersed in Piranha solution for 5 minutes and washed with distilled water. Then, in order to form a boronate-modified self-assembled monolayer (SAM), the electrode body was dipped in a solution of the Cys-FPBA₂ in DMSO for 2 hours. Then, the electrode was washed with DMSO and ethanol, followed by rinse with distilled water (DDW). Finally, the electrode was stored in phosphate buffered saline (PBS).

### Example 2: glycoprotein measurement electrode by bottom-up method

An electrode body was prepared in the same manner as Example 1, and 5 mM cystamine, 20 mM 3-FPBA and a DMSO solvent were used as in Example 1.

Specifically, cystamine was reacted with the gold electrode such that the amino group was exposed to the electrode surface. Then, 3-FPBA having an aldehyde group capable of reacting with the amino group was reacted with the electrode surface so as to be bound to the electrode surface by a Schiff's base.

### Test Example 1: Measurement of electrochemical signals on the electrodes prepared by conjugation method and bottom-up method

In order to confirm whether the electrodes of Examples 1 and 2 were modified with the BA SAM, 25 µg/ml of glucose oxidase (GOX) was reacted with each of the BA-SAM/Au/Si electrodes, and the intensity of the amplified electrochemical signal of GOX, which appeared by the interaction between the glucose chain of GOX and the cis-diol of BA, was measured. In the measurement, 0.1 mM ferrocene (Fc) solution containing 10 mM glucose as a substrate for GOX was used as an electron transmitter. Also, the electrochemical measurement was carried out using cyclic voltammetry at a scan rate of 5 mv/s.

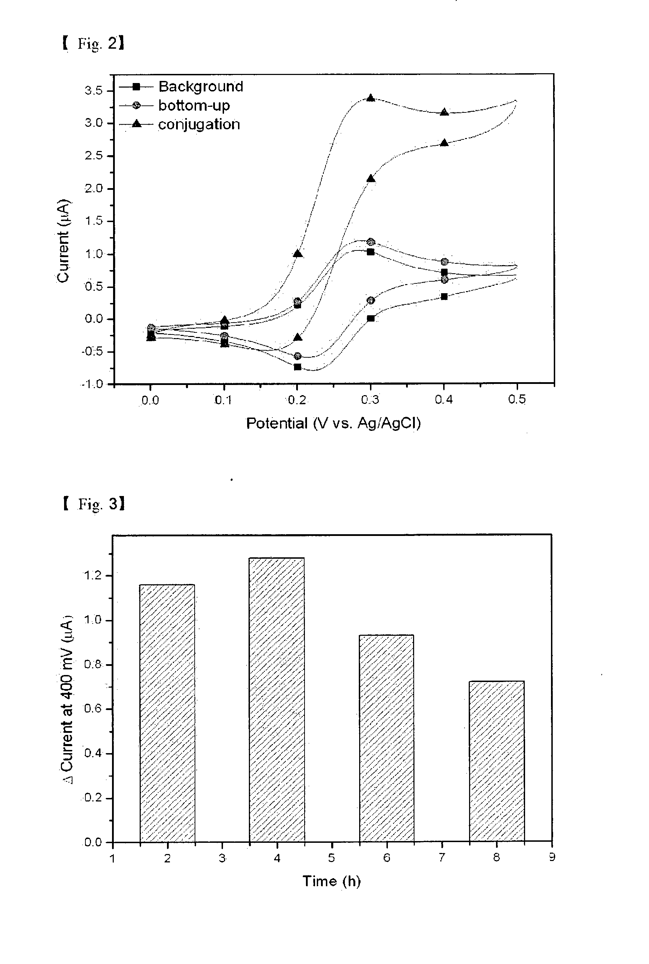

FIG. 2 shows the values of electrochemical signals on the electrodes prepared by the bottom-up method (●, red line) and the conjugation method (▲, blue line) according to the examples of the present invention. As can be seen in FIG. 2, in the BA SAM formed by the conjugation method, the signal increased by about 2 µA compared to the background signal having no substrate. This is attributable to GOX immobilized on the gold electrode surface by the interaction between BA and the cis-diol of GOX, suggesting that the gold electrode surface was modified with BA. However, in the case of the bottom-up method, the signal increased by about 0.3 µA, suggesting that the degree of modification of the electrode surface with BA was not substantially lower than that in the conjugation method.

Thus, it could be seen that the conjugation method of forming a BA self-assembled monolayer (SAM) using a synthesized Cys-FPBA₂ conjugate is significantly effective compared to the bottom-up method. Thus, all subsequent tests were carried out using BA-SAM/Au/Si electrodes obtained by the conjugation method.

### Example 3: Preparation of electrodes using Cys-FPBA₂ synthesized by reacting Cys with FPBA₂ for various times, and measurement of electrochemical signals

In order to determine the optimal reaction time for the synthesis of Cys-FPBA₂, the reaction for synthesis of Cys-FPBA₂ was carried out for various times between 2 hours and 8 hours, and the degree of synthesis of Cys-FPBA₂ was analyzed as a function of reaction time. As described in Example 1, the synthesis of Cys-FPBA₂ was performed by reacting 5 mM cystamine with 20 mM 3FPBA in DMSO. Also, the degree of modification with BA SAM as a function of the synthesis yield and reaction time was examined using a GOX immobilization test in the same manner as Example 1 (BA SAM modification test). The results of the electrochemical test as a function of synthesis time are shown in FIG. 3.

As can be seen in FIG. 3, up to 4 hours, the signal of GOX increased as reaction time increased. However, after 4 hours, the reaction yield decreased slowly, and thus a period of 4 hours was selected as the optimal reaction time. Thus, in subsequent tests, a Cys-FPBA₂ conjugate synthesized by reacting 5 mM cystamine with 20 mM 3FPBA in DMSO for 4 hours was used.

### Example 4: Preparation of electrodes using Cys-FPBA₂ synthesized in the presence of various catalysts, and measurement of electrochemical signals

In order to make sensitive HbA1c measurement possible, the yield of Cys-FPBA₂ that is synthesized should be maximized. In order to more efficiently synthesize Cys-FPBA₂ with high yield, catalysts were used. Based on the results of Example 1, HCl and triethylamine (TEA) were selected as catalysts, and in a control, no catalyst was used. With reference to catalysts previously used in Schiff base synthesis reactions by the present inventors, 100 µl of 0.35% HCl was added to Cys-FPBA₂ conjugates, and 1.1 µl of TEA was added to other Cys-FPBA₂ conjugates.

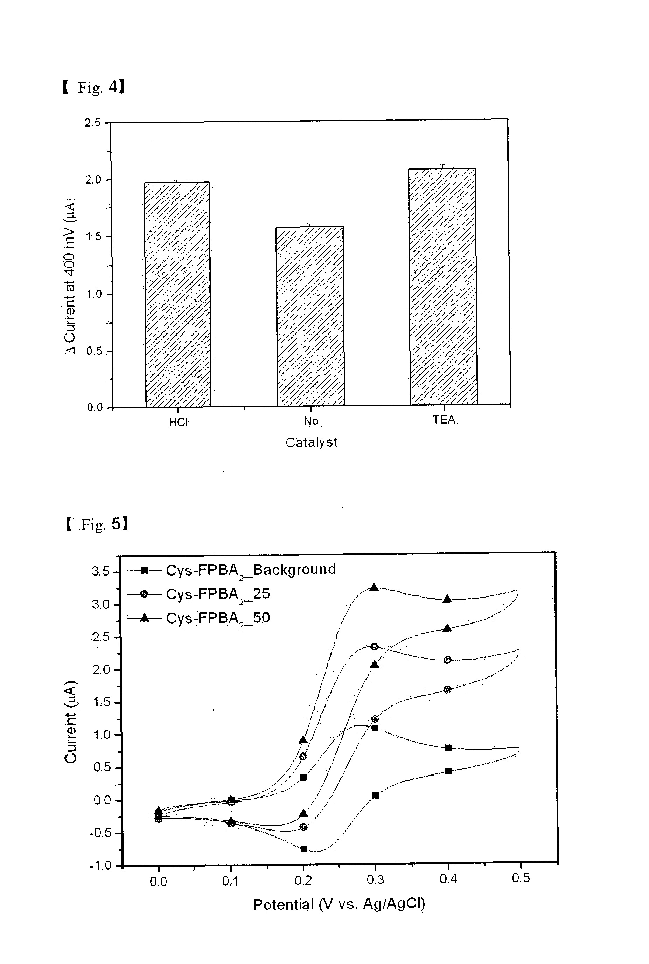

FIG. 4 shows electrochemical signals as a function of the kind of catalyst and shows the current values obtained by subtracting the background signal from the current values at 400 mV on the CV (cyclic voltammogram). As can be seen in FIG. 4, the signal of GOX was higher in the presence of TEA than in the absence of TEA and in the presence of HCl. Thus, it could be seen that the presence of TEA in the synthesis reaction facilitates the synthesis of Cys-FPBA₂. Thus, in subsequent tests, the synthesis of Cys-FPBA₂ was carried out in the presence of 1.1 µl of TEA.

### Example 5: Preparation of electrodes using Cys-FPBA₂ synthesized at various temperatures, and measurement of electrochemical signals

Meanwhile, in order to maximize the synthesis yield of Cys-FPBA₂, the synthesis of Cys-FPBA₂ was carried out at 25 °C (room temperature) and 50 °C based on the results of Example 1, and the results obtained using the Cys-FPBA₂ conjugates synthesized at various temperatures were compared.

FIG. 5 shows the electrochemical signals of GOX immobilized on the Cys-FPBA₂ SAM conjugates synthesized at various temperatures. As can be seen in FIG. 5, the signal of GOX immobilized on the BA SAM synthesized at 50 °C was about two times (about 1 µA) than that on the BA SAM synthesized at 25 °C. This indicates that the amount of GOX immobilized on the Cys-FPBA₂ synthesized at 50 °C was much larger, suggesting that the Cys-FPBA₂ synthesized at 50 °C is significantly efficient. Thus, in subsequent tests, the synthesis of Cys-FPBA₂ was carried out at 50 °C.

### Test Example 2: Examination of formation of Cys-FPBA₂ SAM/Au

Under the optimal conditions for synthesizing Cys-FPBA₂, as determined in Examples 1 to 5 and Test Example 1, 5 mM cystamine and 20 mM 3-formylphenyl boronic acid were reacted with each other in a DMSO solvent at 50 °C for 4 hours in the presence of 1.1 µl of TEA as a catalyst.

In order to confirm whether the surface of Cys-FPBA₂ SAM/Au thus synthesized was modified as desired, a comparative test with cystamine having no BA conjugated thereto was carried out. In the same manner as in the tests in which modification with BA was confirmed, 25 µg/ml of GOX was reacted with each of cystamine having no BA conjugated thereto and a gold electrode having Cys-FPBA₂ immobilized thereon, and the patterns of immobilization with GOX were compared.

It was expected that, in the case of the electrode modified with BA, GOX would be immobilized on the electrode surface by the interaction between BA and cis-diol so that the electrochemical signal of GOX would be detected, and in the case of the cystamine which was not modified with BA, GOX would not be immobilized, because a functional group to which GOX can be immobilized does not exist.

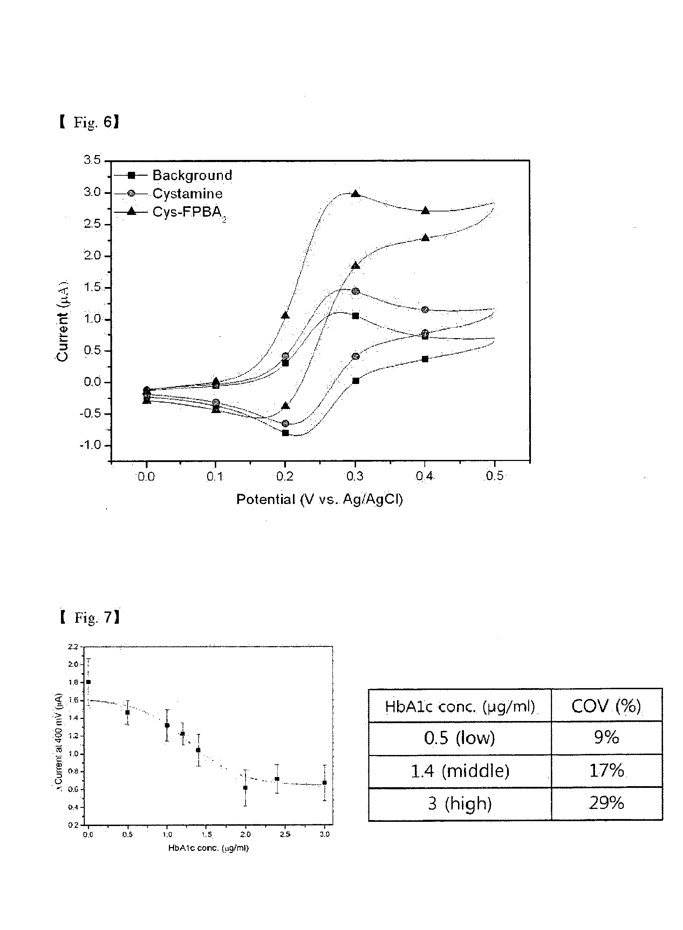

In fact, as can be seen in FIG. 6, in the case of Cys-FPBA₂, the signal increased by about 2.2 µA, but in the case of cystamine, the signal increased by about 0.5 µA. This suggests that, in the case of the Cys-FPBA₂ electrode, GOX was immobilized by specific binding between the *cis*-diol of GOX and the BA group, and in the case of the cystamine, GOX was immobilized by non-specific binding between GOX and the amino group of the cystamine (this binding cannot be used in biosensors, because it is electrostatic binding, is weak and varies depending on the pH of solution). Thus, it could be seen that Cys-FPBA₂ according to the present invention was correctly synthesized so that the electrode surface is modified with the BA group. Using the Cys-FPBA₂ synthesized under the optimal conditions, a BA SAM was formed on the gold electrode surface, and a competition assay for measurement of HbA1c was performed.

### Test Example 3: Electrochemical measurement of HbA1c%

Using Cys-FPBA₂ synthesized under the optimal conditions confirmed in the above examples and test examples, a BA SAM was formed on the electrode surface, and electrochemical measurement was carried out at various concentrations of HbA1c. For electrochemical measurement of HbA1c, native GOX and competition assays were performed.

The competition assay was performed using each of a buffer sample consisting of HbA1c alone (Test Example 3-1), an HbA1c containing BSA (Test Example 3-2), and a reference sample (Test Example 3-3).

### Test Example 3-1: Measurement of HbA1c% in HbA1c sample (in PBS buffer)

In order to determine whether the competition assay of the present invention is suitable for measurement of HbA1c%, a electrochemical signal was detected as a function of the concentration of HbA1c. Specifically, 20 µg/ml of an HbA1c sample (corresponding to a 7500-fold dilution of 150 mg/ml which is the average total hemoglobin concentration of normal persons) was diluted to a concentration ranging from 0% to 15% (0.5 µg/ml to 3 µg/ml), and a signal was measured as function of the concentration of HbA1c.

FIG. 7 shows the values of electrochemical signals and COV as a function of the concentration of HbA1c, measured using the electrode according to the example of the present invention. As can be seen in FIG. 7, as the concentration of HbA1c increased, the electrochemical signal decreased. When the concentration of HbA1c increases, the amount of HbA1c that is immobilized on the electrode surface increases, and thus the binding of GOX capable of generating a signal is spatially limited, and the amount of GOX that binds decreases. Accordingly, as the concentration of HbA1c increases, the electrochemical signal of GOX decreases. As shown in FIG. 7, the signal decreased gradually in the HbA1c concentration range of 2.5-15%, suggesting that the concentration of HbA1c can be electrochemically measured using Cys-FPBA₂ SAM.

In order to verify reproducibility, three dependent repeated tests were carried out. As a result, a low HbA1c concentration of 2.5% (0.5 µg/ml), a middle HbA1c concentration of 7% (1.4 µg/ml) and a high HbA1c concentration of 15% (3 µg/ml) were selected, and COV (coefficient of variation) was calculated. The COV of the standard sample solution consisting of HbA1c alone was 9% in an HbA1c concentration of 2.5% (0.5 µg/ml), 17% in a HbA1c concentration of 7% (1.4 µg/ml), and 29% in a HbA1c concentration of 15% (3 µg/ml), suggesting that COV increases as the concentration of HbA1c increases. This is believed to be because the signal (current) of GOX decreases as the concentration of HbA1c increases, and thus the COV value increases. This test example could demonstrate that the BA SAM-based competition assay is useful for measurement of HbA1c.

### Test Example 3-2: Measurement of HbA1c% in HkaA1c sample (with BSA in PBS buffer)

Because the HbA1c samples in Test Example 3-1 consist of HbA1c alone, the total concentration proteins of the samples which are allowed to react with the electrode modified with GOX differ from each other. The measurement of HbA1c% is carried out to measure HbA1c in a total Hb sample which comprises HbA1c (glycated hemoglobin (Hb)) and HbA0 (non-glycated hemoglobin).

Before a test was carried out in a total Hb sample comprising HbA0 and HbA1c, the signal of HbA1c% comprising HbA1c and BSA in place of HbA0 was measured. Similarly, 20 µg/ml of total protein (assumed as 100% Hb) was diluted to a concentration ranging from 2.5% to 15% to prepare HbA1c samples, and a competition assay of each sample with GOX was carried out.

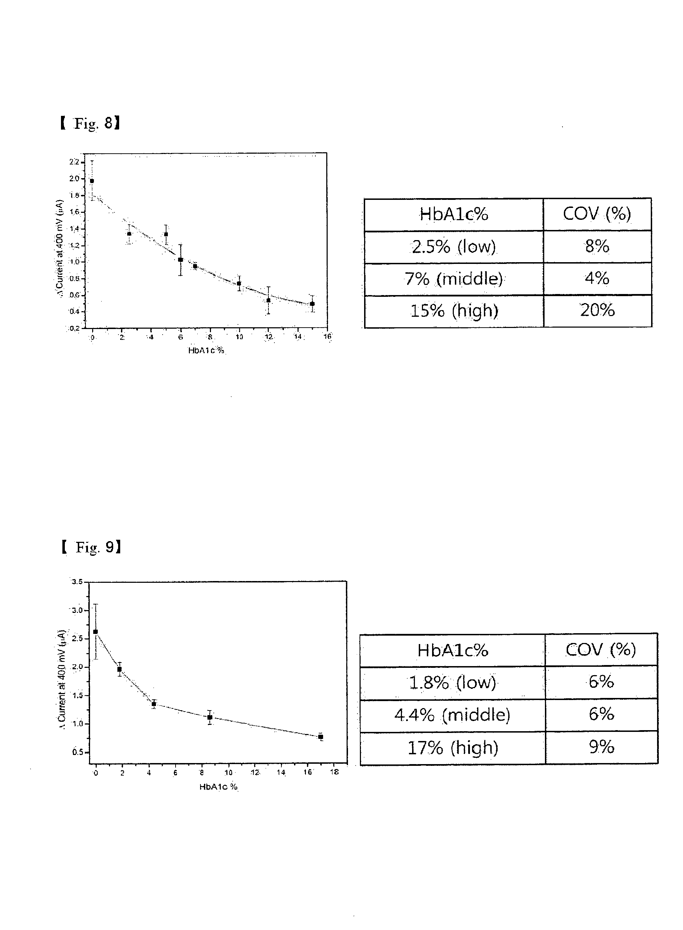

FIG. 8 shows electrochemical signals (current) and COV values as a function of the concentration of HbA1c%, measured using the electrode according to the example of the present invention. As can be seen from the left graph in FIG. 8, as the concentration of HbA1c increased, the electrochemical signal (current) decreased. The right table in FIG. 8 shows COV values obtained by performing three repeated tests in the same manner as the above tests performed using the HbA1c standard solution sample. As can be seen in the right table, the COV values were generally lower than those in Test Example 3-1 performed using HbA1c alone. The reason is as follows. In the case of the sample comprising HbA1c alone, the amount of total protein is not constant, but when the amount of total amount is controlled to a constant level using BSA, the general conditions of the protein sample become uniform, and thus reproducibility is increased.

In this test example, it could be seen that the competition assay can be applied in a test based on total Hb and that the ratio for the Hb sample and the GOX sample for the competition assay is suitable. The ratio of concentration between the two samples is very important in carrying out the competition assay. This is because when the ratio of GOX is too high or low, the electrochemical signal is too large or small to accurately detect the signal of HbA1c.

### Test Example 3-3: Measurement of HbA1c% in HbA1c% sample (reference sample)

In order to measure HbA1c% in blood, a test was carried out using HbA1c reference samples (Lyphochek Hemoglobin Linearity set) purchased from Bio-Rad, Inc. The samples used were reference samples close to whole blood, which were lyophilized according to their HbA1c levels. Because no total hemoglobin level was indicated on the four samples included in the linearity set, the total hemoglobin and glucose levels of each sample were using Reflotron (Roche), before the competition assay was carried out. As a result, it could be seen that the amount of total Hb was different between the samples, and each of the samples contained a significant amount of glucose. Because glucose in blood can react with BA, like HbA1c, it interferes with the measurement of the concentration of HbA1c%. For accurate measurement of HbA1c, glucose was removed from each sample, and then the total Hb of each sample was adjusted to 20 µg/ml and used in the test.

FIG. 9 shows electrochemical signals (current) and COV values as a function of the concentration of %HbA1c in the reference samples, measured using the electrode according to the example of the present invention. Specifically, it shows the results of competition assays carried out using the four reference samples having different HbA1c levels, from which glucose was removed and which were suitably diluted. The sample data sheet show the concentration of HbA1c% in each sample, measured using commercial HbA1c measurement systems. In this Test Example, HbA1c% (IFCC value) measured using Roche/Hitachi Cobas c systems was selected as HbA1c% for each sample. As can be seen in the right data sheet in FIG. 9, samples 1, 2 and 3 had HbA1c concentrations of 1.7%, 4.4% and 17%, respectively.

As shown in FIG. 9, the following five samples were tested: a GOX/BSA sample containing no Hb (HbA1c 0%), as a positive control; and four samples having different HbA1c levels. As can be seen in the left graph, as the concentration of HbA1c% increased, the electrochemical signal (current) in the competition assay decreased. This demonstrates that the competition assay developed in this invention allows the measurement of HbA1c% in blood from which glucose was removed. In addition, the COV value was 6%, suggesting that the samples had high reproducibility compared to other samples.

As described above, according to the present invention, cystamine is bound to the electrode surface, and boronic acid is conjugated to the cystamine. Thus, an increased amount of boronic acid can be bound to the electrode surface, so that the amount of glycoprotein can be more accurately measured using the electrode.

In addition, according to the present invention, the electrode having boronic acid bound thereto can be prepared in a simple and easy manner by preparing a cystamine-boronic acid conjugate and dipping an electrode once in a solution containing the conjugate.

## Claims

1. An electrode for measuring glycoprotein, the electrode comprising:
an electrode body having an electrode surface;
cystamine bound to the electrode surface; and
boronic acid (BA) conjugated to the cystamine;
wherein said boronic acid is bound directly to the cystamine.

2. The electrode of claim 1, wherein the boronic acid is formylphenyl boronic acid (FPBA).

3. The electrode of claim 1, wherein the boronic acid is a 3-formylphenyl boronic acid (3FPBA) represented by the following formula 1:

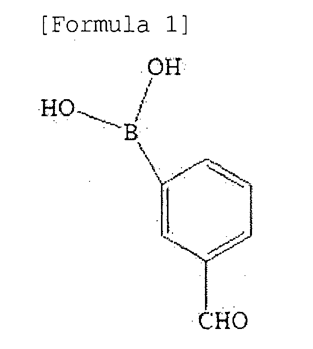

4. The electrode of claim 3, wherein two 3-formylphenyl boronic acids are conjugated to one cystamine.

5. The electrode of any one of claims 1 to 4, wherein the cystamine is bound to the electrode surface by the disulfide group of the cystamine.

6. The electrode of claim 1, wherein a cystamine-boronic acid conjugate prepared by conjugating the cystamine to the boronic acid is bound to the electrode surface.

7. The electrode of claim 6, wherein the conjugate is bound to the electrode surface by the disulfide group of the cystamine.

8. The electrode of claim 6 or 7, wherein the conjugate is a Cys-FPBA₂ represented by the following formula 2:

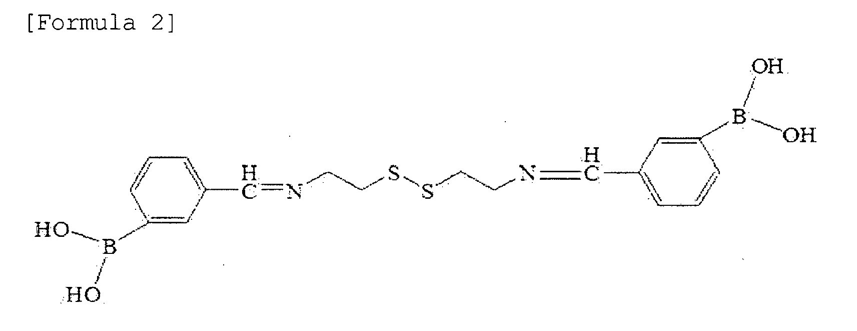

9. The electrode of any one of claims 1 to 8, wherein the electrode surface is made of gold (Au).

10. A method for preparing an electrode for measuring glycoprotein, the method comprising the steps of:
preparing a cystamine-boronic acid conjugate by conjugating cystamine to boronic acid; and
binding the prepared conjugate to an electrode surface
wherein said boronic acid is bound directly to the cystamine.

11. The method of claim 10, wherein the step of preparing the conjugate comprises a step of reacting the cystamine with the boronic acid for 3-5 hours.

12. The method of claim 10 or 11, wherein the step of preparing the conjugate comprises a step of reacting the cystamine with the boronic acid in the presence of triethylamine (TEA).

13. The method of any one of claims 10 to 12, wherein the step of preparing the conjugate comprises a step of reacting the cystamine with the boronic acid at a temperature of 40-60 °C

14. The method of claim 10, wherein the conjugate is a Cys-FPBA₂ represented by the following formula 2:

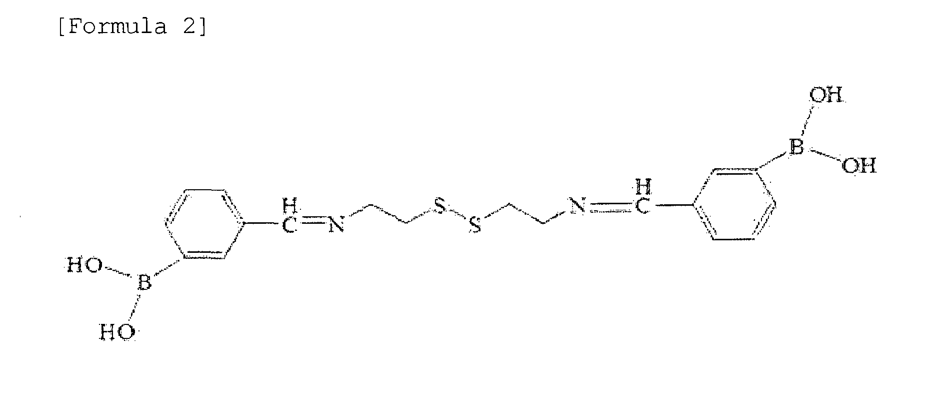

## Patentansprüche

1. Elektrode zum Messen von Glykoprotein, wobei die Elektrode umfasst:
einen Elektrodenkörper mit einer Elektrodenoberfläche;
an die Elektrodenoberfläche gebundenes Cystamin; und
mit dem Cystamin konjugierte Boronsäure (*boronic acid, BA*),
wobei die Boronsäure direkt an das Cystamin gebunden ist.

2. Elektrode nach Anspruch 1, wobei die Boronsäure Formylphenylboronsäure (FPBA) ist.

3. Elektrode nach Anspruch 1, wobei die Boronsäure eine durch die folgende Formel 1 dargestellte 3-Formylphenylboronsäure (3FPBA) ist:

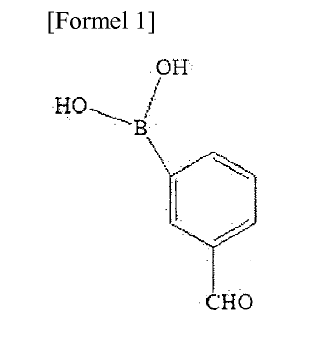

4. Elektrode nach Anspruch 3, wobei zwei 3-Formylphenylboronsäuren mit einem Cystamin konjugiert sind.

5. Elektrode nach einem der Ansprüche 1 bis 4, wobei das Cystamin über die Disulfidgruppe des Cystamins an die Elektrodenoberfläche gebunden ist.

6. Elektrode nach Anspruch 1, wobei ein durch Konjugieren des Cystamins mit der Boronsäure hergestelltes Cystamin-Boronsäure-Konjugat an die Elektrodenoberfläche gebunden ist.

7. Elektrode nach Anspruch 6, wobei das Konjugat über die Disulfidgruppe des Cystamins an die Elektrodenoberfläche gebunden ist.

8. Elektrode nach Anspruch 6 oder 7, wobei das Konjugat ein durch die folgende Formel 2 dargestelltes Cys-FPBA₂-Konjugat ist:

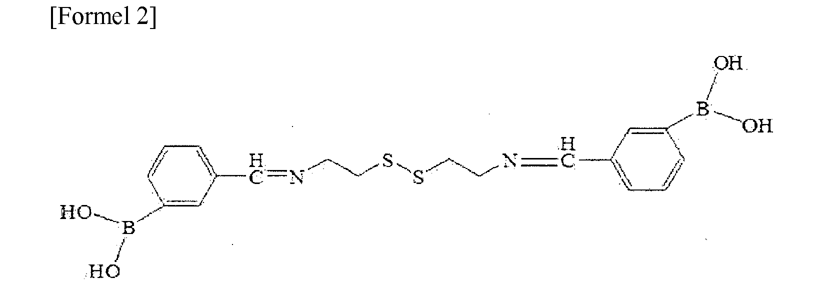

9. Elektrode nach einem der Ansprüche 1 bis 8, wobei die Elektrodenoberfläche aus Gold (Au) gefertigt ist.

10. Verfahren zum Herstellen einer Elektrode zum Messen von Glykoprotein, wobei das Verfahren folgende Schritte umfasst:
Herstellen eines Cystamin-Boronsäure-Konjugats durch Konjugieren von Cystamin mit Boronsäure; und
Binden des hergestellten Konjugats an eine Elektrodenoberfläche,
wobei die Boronsäure direkt an das Cystamin gebunden ist.

11. Verfahren nach Anspruch 10, wobei der Schritt des Herstellens des Konjugats einen Schritt des Zur-Reaktion-Bringens des Cystamins mit der Boronsäure über 3-5 Stunden umfasst.

12. Verfahren nach Anspruch 10 oder 11, wobei der Schritt des Herstellens des Konjugats einen Schritt des Zur-Reaktion-Bringens des Cystamins mit der Boronsäure in Gegenwart von Triethylamin (TEA) umfasst.

13. Verfahren nach einem der Ansprüche 10 bis 12, wobei der Schritt des Herstellens des Konjugats einen Schritt des Zur-Reaktion-Bringens des Cystamins mit der Boronsäure bei einer Temperatur von 40∼60°C umfasst.

14. Verfahren nach Anspruch 10, wobei das Konjugat ein durch die folgende Formel 2 dargestelltes Cys-FPBA₂-Konjugat ist:

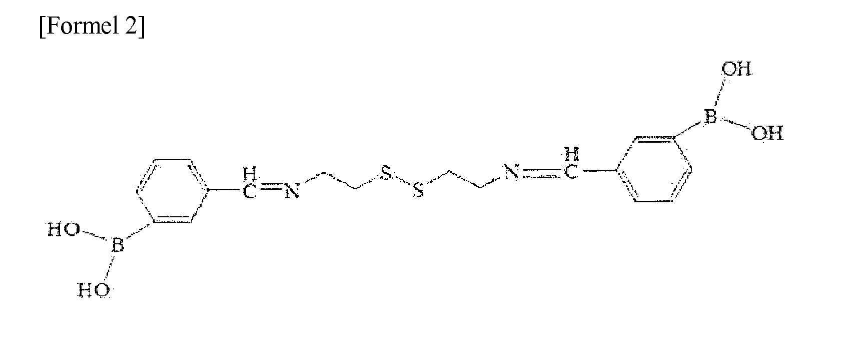

## Revendications

1. Electrode de mesure de glycoprotéine, l'électrode comprenant :
un corps d'électrode ayant une surface d'électrode ;
de la cystamine liée à la surface d'électrode ; et
de l'acide boronique (BA) conjugué à la cystamine ;
dans laquelle ledit acide boronique est lié directement à la cystamine.

2. Electrode selon la revendication 1, dans laquelle l'acide boronique est un acide formylphényl boronique (FPBA).

3. Electrode selon la revendication 1, dans laquelle l'acide boronique est un acide 3-formylphényl boronique (3FPBA) représenté par la formule 1 suivante :

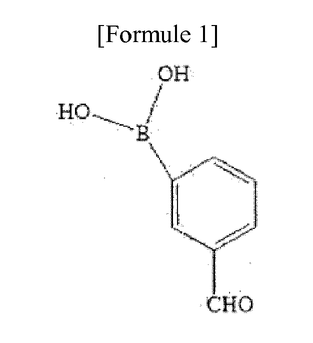

4. Electrode selon la revendication 3, dans laquelle deux acides 3-formylphényl-boroniques sont conjugués à une cystamine.

5. Electrode selon l'une quelconque des revendications 1 à 4, dans laquelle la cystamine est liée à la surface d'électrode par le groupe disulfure de la cystamine.

6. Electrode selon la revendication 1, dans laquelle un conjugué cystamine-acide boronique préparé par la conjugaison de la cystamine à l'acide boronique est lié à la surface d'électrode.

7. Electrode selon la revendication 6, dans laquelle le conjugué est lié à la surface d'électrode par le groupe disulfure de la cystamine.

8. Electrode selon la revendication 6 ou 7, dans laquelle le conjugué est un Cys-FPBA₂ représenté par la formule 2 suivante :

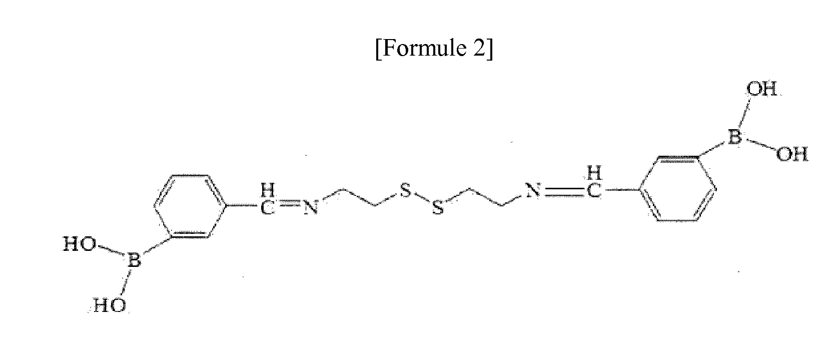

9. Electrode selon l'une quelconque des revendications 1 à 8, dans laquelle la surface d'électrode est faite d'or (Au).

10. Procédé de préparation d'une électrode de mesure de glycoprotéine, le procédé comprenant les étapes suivantes :
la préparation d'un conjugué cystamine-acide boronique par la conjugaison de la cystamine à un acide boronique ; et
la liaison du conjugué préparé à une surface d'électrode,
dans lequel ledit acide boronique est lié directement à la cystamine.

11. Procédé selon la revendication 10, dans lequel l'étape de préparation du conjugué comprend une étape de réaction de la cystamine avec l'acide boronique pendant 3 à 5 heures.

12. Procédé selon la revendication 10 ou 11, dans lequel l'étape de préparation du conjugué comprend une étape de réaction de la cystamine avec l'acide boronique en présence de triéthylamine (TEA).

13. Procédé selon l'une quelconque des revendications 10 à 12, dans lequel l'étape de préparation du conjugué comprend une étape de réaction de la cystamine avec l'acide boronique à une température de 40 °C à 60 °C.

14. Procédé selon la revendication 10, dans lequel le conjugué est un Cys-FPBA₂ représenté par la formule 2 suivante :
